# EUROPEAN PATENT APPLICATION

(11) **EP 4 481 745 A1**
(43) Date of publication of application: **25.12.2024**
(21) Application number: 23756457.0
(22) Date of filing: 16.02.2023
(51) Int. Cl.: G16B 10/00

(54) **METHOD FOR CONDUCTING PHYLOGENETIC ANALYSIS OF CELLS**

(30) Priority: 18.02.2022 JP 2022023985
(71) Applicant: RIKEN, Wako-shi, Saitama 351-0198 (JP)
(72) Inventor: OOTA, Satoshi, Wako-shi, Saitama 351-0198 (JP)
(74) Representative: Mewburn Ellis LLP
(86) International application number: PCT/JP2023/005534
(87) International publication number: WO 2023/157933

(57) **Abstract**

The present invention relates to a method for conducting phylogenetic analysis of cells. The method of the present invention includes steps of: (1) comparing, for a plurality of single cells derived from the same individual, the RNA sequence data of the transcriptome of each single cell with the corresponding genome sequence data, and then screening for a site where the RNA sequence differs from the genome sequence; (2) screening the sites identified by screening in step (1) for a site where three or more single nucleotide polymorphisms are detected, as a site of somatic mutation; and (3) generating a cell lineage tree based on the nucleotide sequence information of the site of somatic mutation obtained in step (2).

## Description

### TECHNICAL FIELD

The present invention relates to a method, a system and a program for conducting phylogenetic analysis of cells.

### BACKGROUND ART

Somatic mutations (mutations of somatic cells) accumulate during the processes of development and aging, causing somatic genome mosaicism. Somatic mutations are associated with various diseases such as dementia, cardiovascular disease, and cancer (NPL 1 and NPL 2) and aging (NPL 3). Furthermore, somatic mutations are also known to be involved in normal development, such as the acquisition of neuronal population diversity in the central nervous system (NPL 4 and NPL 5). In addition, it is known that RNA sequence data analysis reveals clonal expansion of somatic mutations across normal tissues (NPL 6).

During early development of mammals including humans, 2.8 (95% confidence interval, 2.4 to 3.3) somatic mutations occur per cell replication. This is slightly higher than the incidence of mutations in germ cells (germline mutations) (NPL 7 and NPL 8). However, in later stages of development, 6.4 × 10⁻¹⁰ to 7.8 × 10⁻¹⁰ mutations of somatic cells occur per base pair per cell division in various cell types (NPL 8 and NPL 9). The number of mutations of somatic cells can be up to 10 times more than that of germline mutations (NPL 10 and NPL 11). As a result, even monozygotic twins may become genetically diverse due to somatic mutation (NPL 12).

Thus, somatic mutation is an event that occurs in all places and times in various organisms including humans (NPL 13). Somatic mutations can be considered as counterparts to germline mutations (NPL 14), whose dynamics are subject to evolutionary processes (NPL 15). Like germ cells, somatic cells have an "evolutionary record" as "scars" in the genome over time, and their history can theoretically be restored by retrospective cell tracking methods (NPL 16 and NPL 17). Specifically, somatic mutations have the temporal information about cell lineages.

For example, especially in the field of oncology, somatic mutations have been extensively studied in relation to cancer evolution (NPL 18). Researchers have devised mathematical models to elucidate the dynamics of somatic mutations from various biological perspectives (NPL 19 and NPL 20).

However, in the study of cancer evolution, it is necessary to solve multiple difficult problems to detect de novo mutations. The error rate of next-generation sequencing (NGS) technology is very high, making it difficult to detect rare mutations. Solving this difficult problem usually requires ultra-deep sequencing (NPL 21) to improve accuracy. Also, it is necessary to distinguish between true somatic mutations and germline mutations (NPL 22). These challenges, such as high error rates and the need to distinguish somatic mutations from germline mutations for detection, can create noise in the "phylogenetic" features (signature) for estimating ancestral mutations in descendant cells.

Meanwhile, with the advent of single-cell sequencing (SCS), analyses in various fields have become possible, such as rare cell types, uncultured microorganisms, and mosaicization of body tissues (NPL 23).

Since somatic mutations result from alterations in the genome, detection of rare variants with respect to the zygotic reference genome using somatic genome sequence data is a straightforward approach. However, since there are only two copies of each genomic DNA molecule per cell in the case of SCS, SCS has a drawback of causing various technical problems such as ununiform coverage, allelic dropout (ADO) events, false positive (FP) errors and false negative (FN) errors (NPL 24).

Cell type classification and pseudo-time course analysis using machine learning approaches have been developed, such as t-distributed stochastic neighbor embedding (t-SNE) (NPL 32 and NPL 33), and UMAP (uniform manifold approximation and projection) for Dimension Reduction (NPL 34 and NPL 35). However, these techniques may lack biologically relevant interpretation and reproducibility of results, including clustering of low-dimensional data.

### CITATION LIST

### NON PATENT LITERATURE

NPL 1: Kennedy, S. R., Loeb, L. A. & Herr, A. J. Somatic mutations in aging, cancer and neurodegeneration. Mech Ageing Dev 133, 118-126, doi:10.1016/j.mad.2011.10.009 (2012).
NPL 2: Morley, A. A. The somatic mutation theory of ageing. Mutat Res 338, 19-23 (1995).
NPL 3: Kelly, D. P. Ageing theories unified. Nature 470, 342, doi:10.1038/nature09896 (2011).
NPL 4: Abeliovich, A. et al. On somatic recombination in the central nervous system of transgenic mice. Science 257, 404-410 (1992).
NPL 5: McConnell, M. J. et al. Failed clearance of aneuploid embryonic neural progenitor cells leads to excess aneuploidy in the Atm-deficient but not the Trp53-deficient adult cerebral cortex. J Neurosci 24, 8090-8096, doi: 10. 1523/JNEUROSCI.2263-04.2004 (2004).
NPL 6: Yizhak, K. et al. RNA sequence analysis reveals macroscopic somatic clonal expansion across normal tissues. Science 364, eaaw0726, doi:10.1126/science.aaw0726 (2019).
NPL 7: Ju, Y. S. et al. Somatic mutations reveal asymmetric cellular dynamics in the early human embryo. Nature 543, 714-718, doi:10.1038/nature21703 (2017).
NPL 8: Tomasetti, C., Vogelstein, B. & Parmigiani, G. Half or more of the somatic mutations in cancers of self-renewing tissues originate prior to tumor initiation. Proceedings of the National Academy of Sciences 110, 1999, doi: 10.1073/pnas.1221068110 (2013).
NPL 9: Welch, John S. et al. The Origin and Evolution of Mutations in Acute Myeloid Leukemia. Cell 150, 264-278, doi:https://doi.org/10.1016/j.cell.2012.06.023 (2012).
NPL 10: Lynch, M. Rate, molecular spectrum, and consequences of human mutation. Proceedings of the National Academy of Sciences 107, 961, doi:10.1073/pnas.0912629107 (2010).
NPL 11: Van Horebeek, L., Dubois, B. & Goris, A. Somatic Variants: New Kids on the Block in Human Immunogenetics. Trends in Genetics 35, 935-947, doi:https://doi.org/10.1016/j.tig.2019.09.005 (2019).
NPL 12: Jonsson, H. et al. Differences between germline genomes of monozygotic twins. Nature Genetics 53, 27-34, doi:10.1038/s41588-020-00755-1 (2021).
NPL 13: Garcia-Nieto, P. E., Morrison, A. J. & Fraser, H. B. The somatic mutation landscape of the human body. Genome Biology 20, 298, doi:10.1186/s13059-019-1919-5 (2019).
NPL 14: Milholland, B. et al. Differences between germline and somatic mutation rates in humans and mice. Nat Commun 8, 15183, doi: 10.1038/ncomms15183 (2017).
NPL 15: Rozhok, A. I. & DeGregori, J. Toward an evolutionary model of cancer: Considering the mechanisms that govern the fate of somatic mutations. Proc Natl Acad Sci U S A 112, 8914-8921, doi:10.1073/pnas.1501713112 (2015).
NPL 16: Woodworth, M. B., Girskis, K. M. & Walsh, C. A. Building a lineage from single cells: genetic techniques for cell lineage tracking. Nature Reviews Genetics 18, 230, doi:10.1038/nrg.2016.159 (2017).
NPL 17: Oota, S. Somatic mutations - Evolution within the individual. Methods 176, 91-98, doi:https://doi.org/10.1016/j.ymeth.2019.11.002 (2020).
NPL 18: McGranahan, N. & Swanton, C. Clonal Heterogeneity and Tumor Evolution: Past, Present, and the Future. Cell 168, 613-628, doi:10.1016/j.cell.2017.01.018 (2017).
NPL 19: Beerenwinkel, N., Schwarz, R. F., Gerstung, M. & Markowetz, F. Cancer Evolution: Mathematical Models and Computational Inference. Systematic Biology 64, e1-e25, doi:10.1093/sysbio/syu081 (2015).
NPL 20: Altrock, P., Liu, L. & Michor, F. The mathematics of cancer: Integrating quantitative models. Nature Reviews Cancer 15, 730-745, doi:10.1038/nrc4029 (2015).
NPL 21: Rheinbay, E. et al. Recurrent and functional regulatory mutations in breast cancer. Nature 547, 55-60, doi:10.1038/nature22992 (2017).
NPL 22: Sun, J. X. et al. A computational approach to distinguish somatic vs. germline origin of genomic alterations from deep sequencing of cancer specimens without a matched normal. PLoS Comput Biol 14, e1005965, doi:10.1371/journal.pcbi.1005965 (2018).
NPL 23: Method of the year 2013. Nat Methods 11, 1, doi:10.1038/nmeth.2801 (2014).
NPL 24: Wang, Y. & Navin, N. E. Advances and applications of single-cell sequencing technologies. Mol Cell 58, 598-609, doi:10.1016/j.molcel.2015.05.005 (2015).
NPL 25: Sheng, Q., Zhao, S., Li, C. I., Shyr, Y. & Guo, Y. Practicability of detecting somatic point mutation from RNA high throughput sequencing data. Genomics 107, 163-169, doi:10.1016/j.ygeno.2016.03.006 (2016).
NPL 26: Tam, P. P. L. & Ho, J. W. K. Cellular diversity and lineage trajectory: insights from mouse single cell transcriptomes. Development 147, dev179788, doi:10.1242/dev.179788 (2020).
NPL 27: Ji, Z. & Ji, H. TSCAN: Pseudo-time reconstruction and evaluation in single-cell RNA-seq analysis. Nucleic acids research 44, e117-e117, doi:10.1093/nar/gkw430 (2016).
NPL 28: Hou, W. et al. A statistical framework for differential pseudotime analysis with multiple single-cell RNA-seq samples. bioRxiv : the preprint server for biology, 2021.2007.2010.451910, doi:10.1101/2021.07.10.451910 (2021).
NPL 29: Campbell, K. R. & Yau, C. Uncovering pseudotemporal trajectories with covariates from single cell and bulk expression data. Nature communications 9, 2442-2442, doi:10.1038/s41467-018-04696-6 (2018).
NPL 30: Felsenstein, J. The Number of Evolutionary Trees. Systematic Biology 27, 27-33, doi:10.2307/2412810 (1978).
NPL 31: Gott, J. R., III et al. A Map of the Universe. The Astrophysical Journal 624, 463-484, doi:10.1086/428890 (2005).
NPL 32: Hinton, G. & Roweis, S. Stochastic Neighbor Embedding. Advances in neural information processing systems 15, 833--840 (2003).
NPL 33: Maaten, L. v. d. & Hinton, G. Visualizing Data using t-SNE. Journal of Machine Learning Research 9, 2579-2605 (2008).
NPL 34: McInnes, L., Healy, J. & Melville, J. UMAP: Uniform Manifold Approximation and Projection for Dimension Reduction. arXiv, 1802.03426 (2020).
NPL 35: McInnes, L., Healy, J., Saul, N. & Grosberger, L. UMAP: Uniform Manifold Approximation and Projection. Journal of Open Source Software 3, 861 (2018).
NPL 36: Pavlicev, M. et al. Single-cell transcriptomics of the human placenta: inferring the cell communication network of the maternal-fetal interface. Genome Res 27, 349-361, doi:10.1101/gr.207597.116 (2017).
NPL 37: Sanchez, C., et al. Grasping at molecular interactions and genetic networks in Drosophila melanogaster using FlyNets, an Internet database. Nucleic acids research 27, 89-94, doi:10.1093/nar/27.1.89 (1999).
NPL 38: Schneider, V. A. et al. Evaluation of GRCh38 and de novo haploid genome assemblies demonstrates the enduring quality of the reference assembly. bioRxiv, 072116, doi:10.1101/072116 (2016).
NPL 39: Church, D. M. et al. Modernizing reference genome assemblies. PLoS Biol 9, e1001091, doi:10.1371/journal.pbio.1001091 (2011).
NPL 40: Li, H. & Durbin, R. Fast and accurate short read alignment with Burrows-Wheeler transform. Bioinformatics 25, 1754-1760, doi:10.1093/bioinformatics/btp324 (2009).
NPL 41: Bolger, A. M., Lohse, M. & Usadel, B. Trimmomatic: a flexible trimmer for Illumina sequence data. Bioinformatics 30, 2114-2120, doi:10.1093/bioinformatics/btu170 (2014).
NPL 42: Li, H. et al. The Sequence Alignment/Map format and SAMtools. Bioinformatics 25, 2078-2079, doi:10.1093/bioinformatics/btp352 (2009).
NPL 43: Kimura, M. The number of heterozygous nucleotide sites maintained in a finite population due to steady flux of mutations. Genetics 61, 893-903 (1969).
NPL 44: Tajima, F. Infinite-allele model and infinite-site model in population genetics. Journal of Genetics 75, 27, doi:10.1007BF02931749 (1996).
NPL 45: Cingolani, P. et al. A program for annotating and predicting the effects of single nucleotide polymorphisms, SnpEff: SNPs in the genome of Drosophila melanogaster strain w1118; iso-2; iso-3. Fly (Austin) 6, 80-92, doi:10.4161/fly.19695 (2012).
NPL 46: Farris, J. S. Methods for Computing Wagner Trees. Systematic Biology 19, 83-92, doi:10.1093/sysbio/19.1.83 (1970).
NPL 47: Fitch, W. M. Toward Defining the Course of Evolution: Minimum Change for a Specific Tree Topology. Systematic Zoology 20, 406-416, doi:10.2307/2412116 (1971).
NPL 48: Tamura, K., Stecher, G. & Kumar, S. MEGA11: Molecular Evolutionary Genetics Analysis Version 11. Molecular Biology and Evolution 38, 3022-3027, doi:10.1093/molbev/msab120 (2021).
NPL 49: Felsenstein, J. PHYLIP - Phylogeny Inference Package (Version 3.2). Cladistics 5, 164-166 (1989).
NPL 50: Yadav, V. K., DeGregori, J. & De, S. The landscape of somatic mutations in protein coding genes in apparently benign human tissues carries signatures of relaxed purifying selection. Nucleic Acids Res 44, 2075-2084, doi:10.1093/nar/gkw086 (2016).
NPL 51: Persi, E., Wolf, Y. I., Leiserson, M. D. M., Koonin, E. V. & Ruppin, E. Criticality in tumor evolution and clinical outcome. Proceedings of the National Academy of Sciences 115, E11101-E11110, doi:10.1073/pnas.1807256115 (2018).
NPL 52: Yang, Z. PAML 4: phylogenetic analysis by maximum likelihood. Mol Biol Evol 24, 1586-1591, doi:10.1093/molbev/msm088 (2007).
NPL 53: Nei, M. & Gojobori, T. Simple methods for estimating the numbers of synonymous and nonsynonymous nucleotide substitutions. Mol Biol Evol 3, 418-426, doi: 10. 1 093/oxfordj ournals.molbev.a04041 0 (1986).
NPL 54: R Core Team. R: A Language and Environment for Statistical Computing (Vienna, Austria, 2016).
NPL 55: Blondel, V. D., Guillaume, J.-L., Lambiotte, R. & Lefebvre, E. Fast unfolding of communities in large networks. Journal of Statistical Mechanics: Theory and Experiment 2008, P10008, doi:10.1088/1742-5468/2008/10/p10008 (2008).
NPL 56: Trapnell, C. et al. The dynamics and regulators of cell fate decisions are revealed by pseudotemporal ordering of single cells. Nature Biotechnology 32, 381-386, doi:10.1038/nbt.2859 (2014).
NPL 57: Qiu, X. et al. Reversed graph embedding resolves complex single-cell trajectories. Nature Methods 14, 979-982, doi:10.1038/nmeth.4402 (2017).
NPL 58: Cao, J. et al. The single-cell transcriptional landscape of mammalian organogenesis. Nature 566, 496-502, doi:10.1038/s41586-019-0969-x (2019).
NPL 59: Mathematica (Wolfram Research, Inc., Champaign, Illinois, 2020).
NPL 60: Phylogenetics for Mathematica. Version 6.5 (Indiana University: Bloomington, Indiana, Department of Earth and Atmospheric Sciences, 2019).
NPL 61: Zachar, I. IstvanZachar/Phylogenetics, <https://github.com/IstvanZachar/Phylogenetics/releases/tag/1.1.0> (2017).
NPL 62: Archie, J. et al. The Newick tree format, <https://evolution.genetics.washington.edu/phylip/newicktree.html> (1986).
NPL 63: Kumar, A. An overview of nested genes in eukaryotic genomes. Eukaryot Cell 8, 1321-1329, doi:10.1128/EC.00143-09 (2009).
NPL 64: Long, C. A. Sokal, Robert R., and Peter H. A. Sneath. Principles of Numerical Taxonomy. W. H. Freeman and Co., San Francisco and London. Pp. xvi + 359, illus. 1963. Price $8.50. Journal of Mammalogy 46, 111-112, doi:10.2307/1377831 (1965).
NPL 65: Knofler, M. et al. Human placenta and trophoblast development: key molecular mechanisms and model systems. Cellular and Molecular Life Sciences 76, 3479-3496, doi:10.1007/s00018-019-03104-6 (2019).
NPL 66: Yang, Z., Wong, W. S. W. & Nielsen, R. Bayes Empirical Bayes Inference of Amino Acid Sites Under Positive Selection. Molecular Biology and Evolution 22, 1107-1118, doi:10.1093/molbev/msi097 (2005).
NPL 67: Zhang, T., Periz, G., Lu, Y. N. & Wang, J. USP7 regulates ALS-associated proteotoxicity and quality control through the NEDD4L-SMAD pathway. Proc Natl Acad Sci U S A 117, 28114-28125, doi:10.1073/pnas.2014349117 (2020).
NPL 68: Bittoni, A. et al. Retrospective Cohort Study of Caveolin-1 Expression as Prognostic Factor in Unresectable Locally Advanced or Metastatic Pancreatic Cancer Patients. Curr Oncol 28, 3525-3536, doi:10.3390/curroncol28050303 (2021).
NPL 69: Kobak, D. & Berens, P. The art of using t-SNE for single-cell transcriptomics. Nature Communications 10, 5416, doi:10.1038/s41467-019-13056-x (2019).
NPL 70: Ortega, M. A. et al. Using single-cell multiple omics approaches to resolve tumor heterogeneity. Clinical and Translational Medicine 6, 46, doi:10.1186/s40169-017-0177-y (2017).
NPL 71: Araten, D. J. et al. A Quantitative Measurement of the Human Somatic Mutation Rate. Cancer Research 65, 8111-8117, doi:10.1158/0008-5472.Can-04-1198 (2005).
NPL 72: Dou, Y., Gold, H. D., Luquette, L. J. & Park, P. J. Detecting Somatic Mutations in Normal Cells. Trends Genet 34, 545-557, doi:10.1016/j.tig.2018.04.003 (2018).
NPL 73: Rhee, J.-K., Lee, S., Park, W.-Y., Kim, Y.-H. & Kim, T.-M. Allelic imbalance of somatic mutations in cancer genomes and transcriptomes. Scientific Reports 7, 1653, doi:10.1038/s41598-017-01966-z (2017).
NPL 74: Ju, Y. S. et al. Extensive genomic and transcriptional diversity identified through massively parallel DNA and RNA sequencing of eighteen Korean individuals. Nature Genetics 43, 745-752, doi:10.1038/ng.872 (2011).
NPL 75: Neums, L. et al. VaDiR: an integrated approach to Variant Detection in RNA. Gigascience 7, 1-13, doi:10.1093/gigascience/gix122 (2018).
NPL 76: Browning, S. R. & Browning, B. L. Haplotype phasing: existing methods and new developments. Nature reviews. Genetics 12, 703-714, doi:10.1038/nrg3054 (2011).
NPL 77: Wasik, K. et al. Comparing low-pass sequencing and genotyping for trait mapping in pharmacogenetics. BMC Genomics 22, 197, doi:10.1186/s12864-021-07508-2 (2021).
NPL 78: Cock, P. J. A., Fields, C. J., Goto, N., Heuer, M. L. & Rice, P. M. The Sanger FASTQ file format for sequences with quality scores, and the Solexa/Illumina FASTQ variants. Nucleic acids research 38, 1767-1771, doi:10.1093/nar/gkp1137 (2010).
NPL 79: Danecek, P. et al. The variant call format and VCFtools. Bioinformatics (Oxford, England) 27, 2156-2158, doi:10.1093/bioinformatics/btr330 (2011).

### SUMMARY OF INVENTION

### TECHNICAL PROBLEM

As a result of diligent research to solve the above problems, the present inventors have found that phylogenetic analysis of cells can be conducted by focusing on somatic mutations identified by comparing sequence data of the transcriptome with reference genome sequence data, and thus conceived the present invention.

The present invention focuses on phylogenetic signatures of single-cell transcriptomes. For example, 60 somatic cells have about 10⁹⁶ possible lineages, and this is greater than the number of atoms existing in the universe (NPL 31). The method of the present invention takes advantage of the coarse phylogenetic signal retained in RNA sequence data to narrow the window covering true lineages. Unlike single-cell sequencing (SCS), hundreds or thousands of copies of intracellular RNA molecules can be used for the source of RNA sequences. False positives due to biological and technical factors such as RNA editing, sequencing errors (e.g., random errors that occur during reverse transcription and PCR), and potential sampling errors should be taken into account (NPL 13 and NPL 25). However, single-cell transcriptome data are overwhelmingly abundant and compensate for these disadvantages of RNA sequencing data (NPL 26).

### SOLUTION TO PROBLEM

The present invention includes, but is not limited to, the following aspects.
[1] A method for conducting phylogenetic analysis of cells, including steps of:
   (1) comparing, for a plurality of single cells derived from the same individual, the RNA sequence data of the transcriptome of each single cell with the corresponding genome sequence data, and then screening for a site where the RNA sequence differs from the genome sequence;
   (2) screening the sites identified by screening in step (1) for a site where three or more single nucleotide polymorphisms are detected, as a site of somatic mutation; and
   (3) generating a cell lineage tree based on the nucleotide sequence information of the site of somatic mutation obtained in step (2).
[2] The method according to [1], further including a step of (4) estimating the cell type of each single cell from the cell lineage tree.
[3] The method according to [1] or [2], further including a step of comparing the information of the single cell type estimated from the gene expression profile of each single cell with the information of the cell lineage tree.
[4] The method according to any one of [1] to [3], wherein the single nucleotide polymorphism is a single nucleotide substitution.
[5] The method according to any one of [1] to [4], wherein in step (1), a site, for which the RNA sequence data of the transcriptome cannot be obtained in 50% or more of all cells, is presumed to have no mutation and is excluded from screening.
[6] The method according to any one of [1] to [5], wherein the somatic mutation to be screened for in step (2) is a low frequent somatic mutation.
[7] The method of any one of [1] to [6], for detecting a somatic mutation associated with a disease or a symptom.
[8] The method according to [7], wherein the disease or the symptom is selected from the group consisting of cancer, dementia, cardiovascular disease, aging, autoimmune disease, neurodegenerative disease, and psychiatric disease.
[9] A program for performing the method according to any one of [1] to [8].
[10] A storage medium, on which a program for performing the method according to any one of [1] to [8] is stored.
[11] A system including a processor and a memory having a program stored therein that performs the method according to any one of [1] to [8] when the program is executed by the processor.

### ADVANTAGEOUS EFFECTS OF INVENTION

The method of the present invention utilizes potential somatic mutations detected from single-cell transcriptome data to provide a new framework for detecting cell lineage trajectories. Unlike pseudo-time course analysis that involves analyzing heterogeneously differentiated cells in a single time snapshot, the present invention is capable of tracing cell lineages back to ancestral cells using somatic mutations.

### BRIEF DESCRIPTION OF DRAWINGS

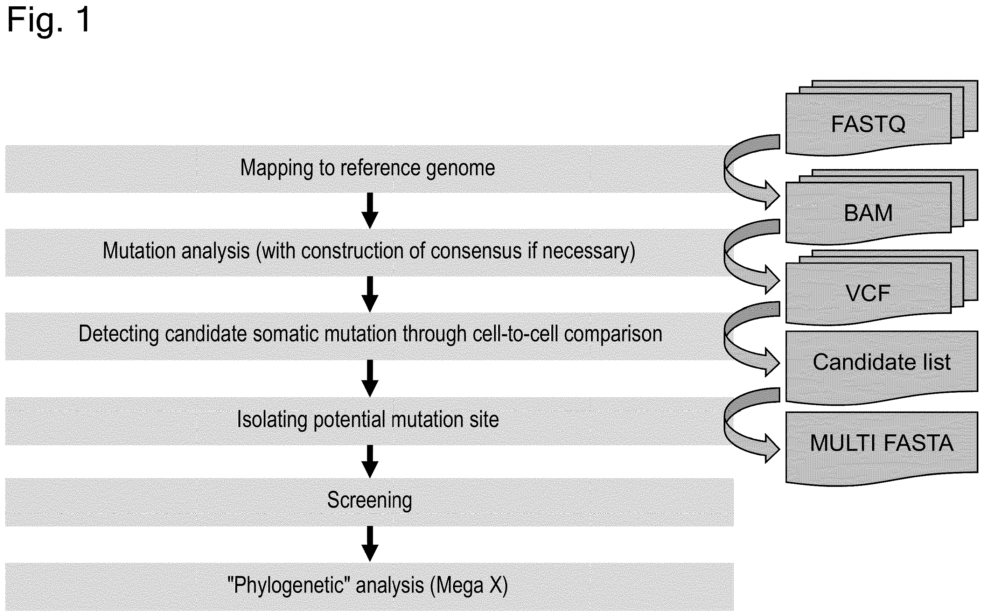

Fig. 1 depicts a workflow for analyzing the somatic mutation patterns of single cells. FASTQ (NPL 78), BAM (NPL 4) and VCF (NPL 79) files were generated for each single cell. FASTQ, BAM, and VCF are data formats for describing genetic information.

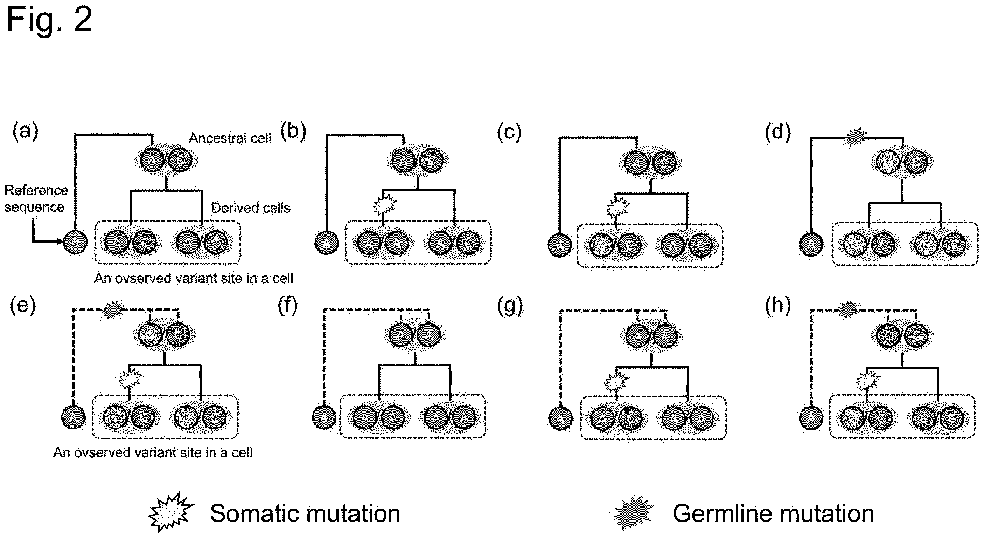

Fig. 2 depicts detection of a somatic mutation with the use of a variant with a multiple mutation site. (a) An apparent mutation site derived from an ancestral heterozygous site with no germline or somatic mutations. (b) An apparent mutation site derived from an ancestral heterozygous site with a somatic mutation. The mutational context of this cell cannot be distinguished from (a). (c) A mutation site derived from an ancestral heterozygous site with a somatic mutation. The mutational context of this cell is distinguishable from (a) and (b) based on a third type of nucleotide G. (d) An apparent mutation site derived from an ancestral heterozygous site with a germline mutation. The mutational context of this cell cannot be distinguished from (h). (e) A mutation site derived from an ancestral heterozygous site with a germline mutation followed by a somatic mutation. The mutational context of this cell is distinguishable from (d) and (h) based on a third type of nucleotide T. (f) A homo site derived from a homozygous site. (g) A mutation site derived from an ancestral heterozygous site with a somatic mutation. The mutational context of this cell cannot be distinguished from (a) and (b). (h) An apparent mutation site derived from an ancestral heterozygous site with a germline mutation followed by a somatic mutation. The mutational context of this cell cannot be distinguished from (d). In Fig. 2, the reference genome was determined to be in a haplotype phase for the sake of simplicity. Solid lines indicate reference sites in the haplotype phase. Dotted lines indicate homozygous reference sites. This figure exemplarily represents two typical cells derived from ancestral cells.

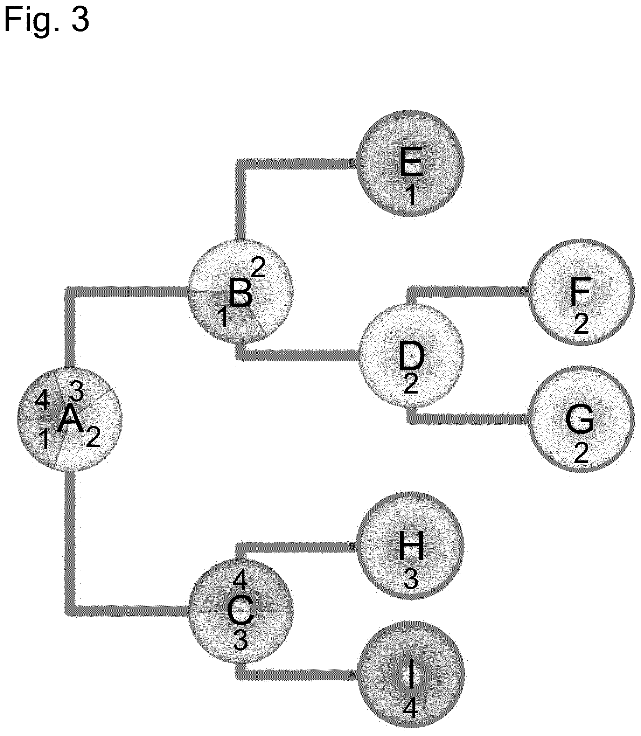

Fig. 3 depicts the evaluation of the degree of monophyleticity (DoM) at each node as the percentage of clusters in the subtree. The gene expression profiles (table of clusters) are mapped in the form of a tree topology. If the DoM and gene expression profiles match perfectly, a set of subtrees can be observed, which is monophyletic in terms of clusters. If the DoM and gene expression profiles do not match perfectly, paraphyletic subtrees or multiphyletic subtrees will be observed in terms of clusters. In this way, the consistency of the size of a monophyletic subtree with respect to the total number of subtrees can be evaluated. The percentage of the number of cells assigned to a cluster is calculated with the use of Mathematica (NPL 59) code, AssignCluster2Cell, so that the monophyleticity of DoM of the subtree can be evaluated. A: Ancestral stem cells; B, C and D: stem cell-derived cells; E, F, G and I: observed differentiated single cells.

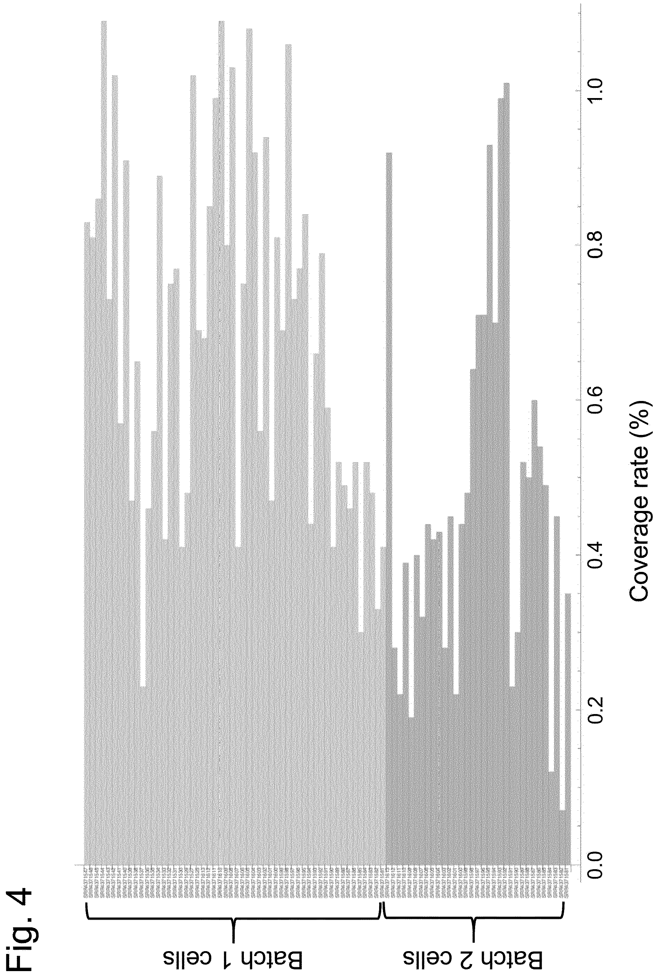

Fig. 4 depicts the coverage rate (%) of the transcriptome mapped to the reference genome (GRCh38). There was a large difference in coverage among the cells. Horizontal axis: coverage rate (%) in the reference genome; vertical axis: name of each single cell in batch 1 and batch 2 sampled from 2 pregnant women.

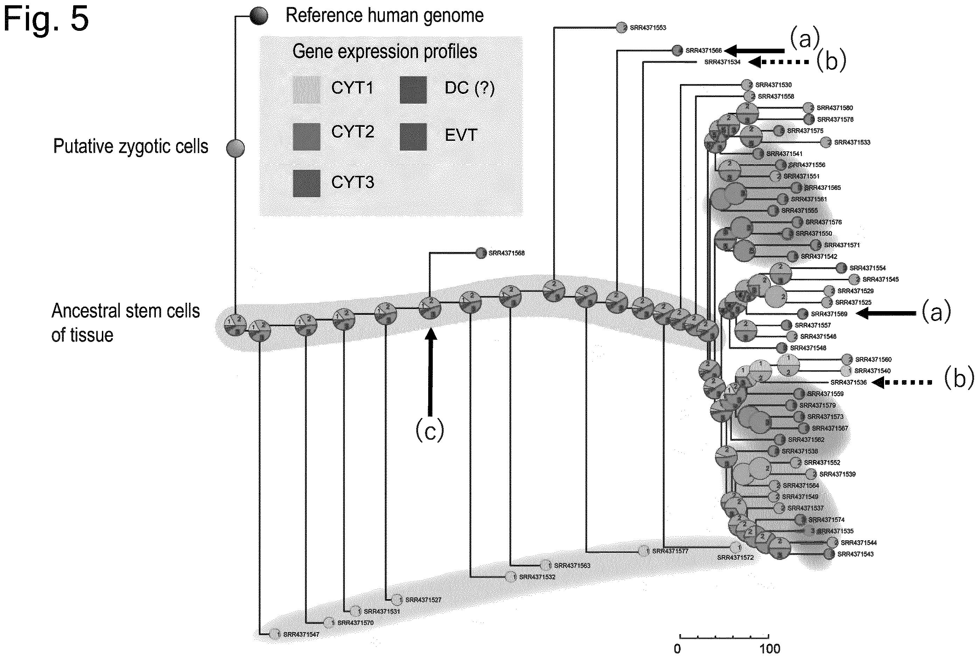

Fig. 5 is the mapping of gene expression profiles to a cell lineage tree (SRP090944 data; batch 1, 54 cells). Based on a study by Pavlicev et al. (NPL 36), single-cell data were classified into five clusters (cell types), cytotrophoblast (CYT) 1, CYT2, CYT3, extravillous trophoblast (EVT), and maternal decidual cells (DC) and mapped in a tree topology, using gene expression profiles and through principal component analysis (PCA) on 300 marker genes . (a) Placental cells, although reported as maternal decidual cells (DC) in the previous study by Pavlicev et al. (NPL 36); (b) non annotated placental cells (NPL 36); and (c) putative stem cells in the self-renewal stage. Scale bar: expected values of mutations. Each numerical value in a pie chart represents a specified cluster based on the gene expression profile.

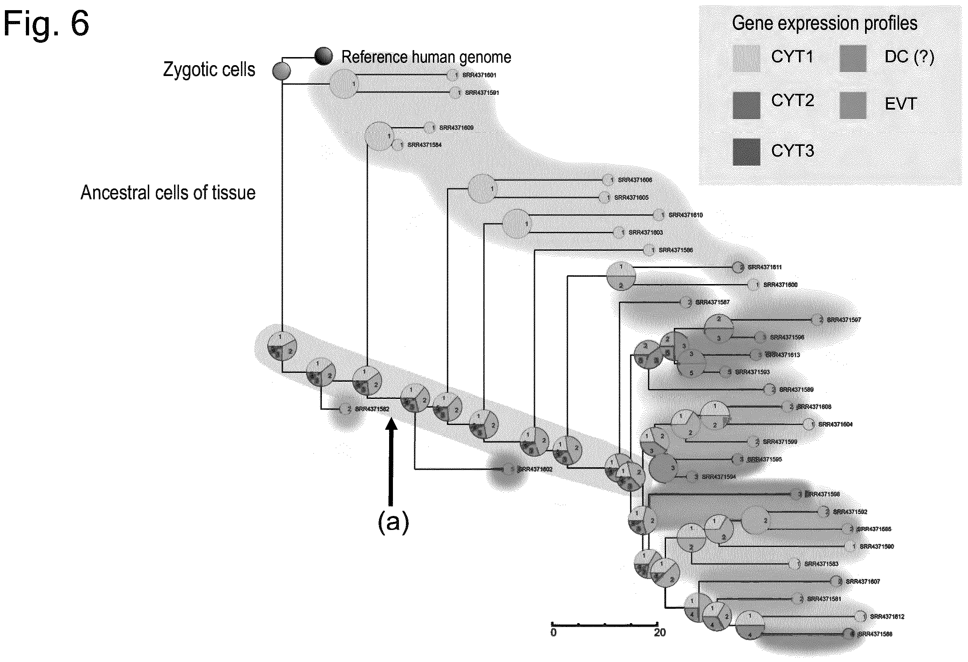

Fig. 6 is the mapping of gene expression profiles to a cell lineage tree (SRP090944 data; batch 2, 23 cells). (a) Putative stem cells in the self-renewal stage. Other notations are the same as those in Fig. 5.

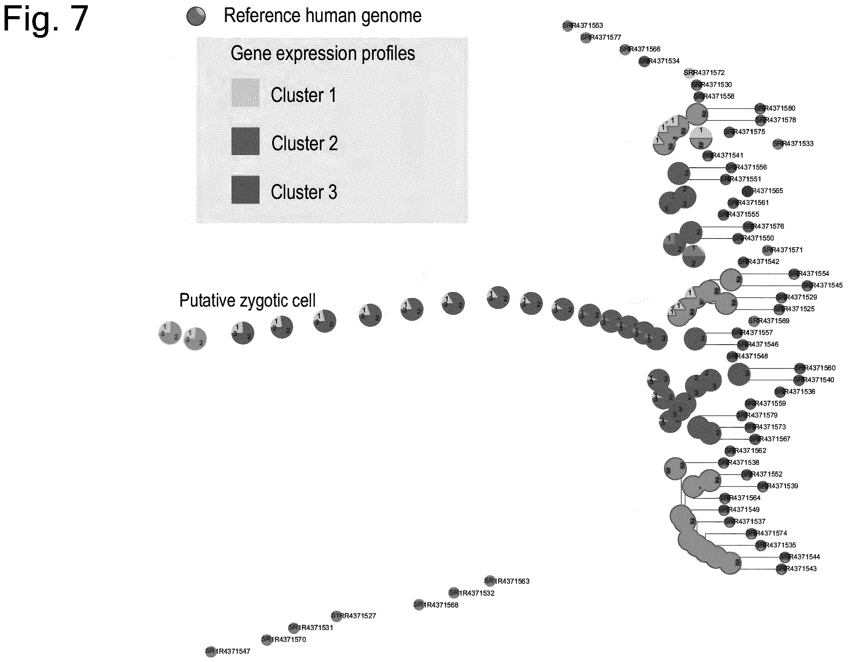

Fig. 7 is the mapping of gene expression profiles re-analyzed by t-SNE to a cell lineage tree (SRP090944 data; batch 1, 54 cells).

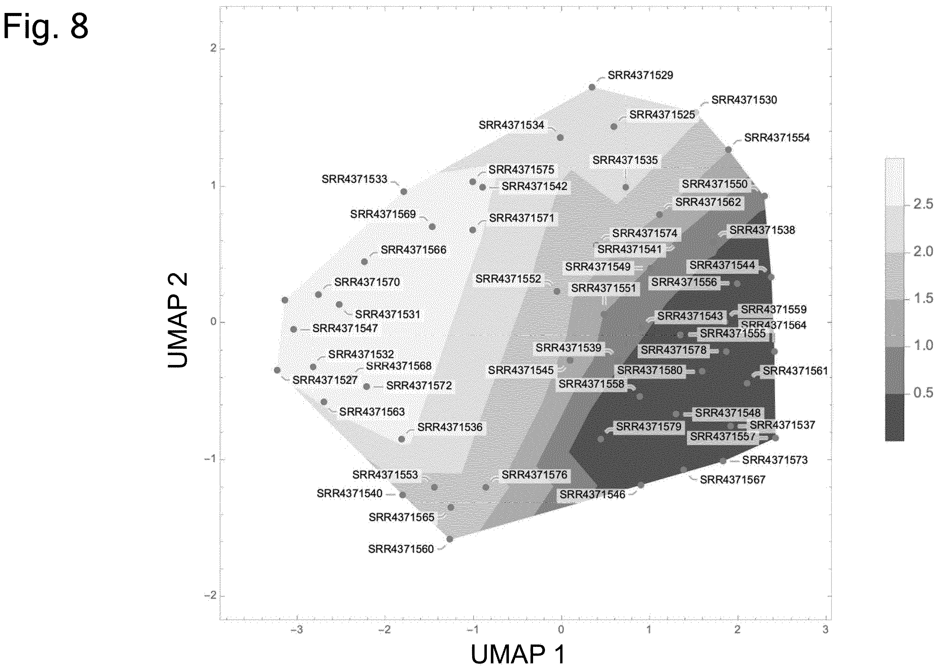

Fig. 8 depicts the results of a pseudo-time course analysis of SRP090944 data; batch 1. Contours were generated by Mathematica ListContourPlot function (NPL 59) with an interpolation order of 3. The figure indicates pseudo-time in color. Not all cells are labeled in this figure because of the Mathematica algorithm.

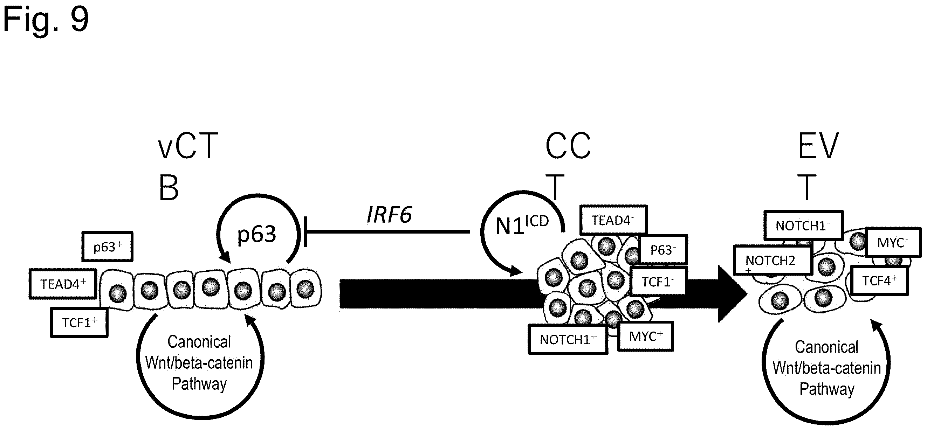

Fig. 9 relates to a model system linking the function of Notch/Wnt signals to cytotrophoblast and extravillous trophoblast differentiation (NPL 65). vCTB: villous cytotrophoblast; CCT: cell column trophoblast; EVT: extravillous trophoblast; N1^{ICD}: Notch1 intercellular domain; TEAD4: transcription enhancer factor TEF-3 gene; p63: neoplasm protein p63; TCF1: transcription factor T-cell factor 1; TCF4: transcription factor T-cell factor 4; IRF6: interferon regulatory factor 6; Notch2: Notch receptor 2.

### DESCRIPTION OF EMBODIMENTS

The present invention includes, without limitation, the following aspects. Unless defined otherwise herein, technical and scientific terms used herein have the same meanings as commonly understood by a person skilled in the art. The substances, materials and examples disclosed herein are exemplary only and are not intended to be limiting. When the specification refers to "in one aspect," it is meant to be not limited to that aspect, i.e., non-limiting.

### 1. Method for conducting phylogenetic analysis of cells

In one aspect, the present invention relates to a method for conducting phylogenetic analysis of cells.

The method includes:
(1) comparing, for a plurality of single cells derived from the same individual, the RNA sequence data of the transcriptome of each single cell with the corresponding genome sequence data, and then screening for a site where the RNA sequence differs from the genome sequence;
(2) screening the sites identified by screening in step (1) for a site where three or more single nucleotide polymorphisms are detected, as a site of somatic mutation; and
(3) generating a cell lineage tree based on the nucleotide sequence information of the site of somatic mutation obtained in step (2).

The method of the present invention utilizes a plurality of single cells derived from the same individual. The type of individual is not particularly limited. Examples thereof include, but are not limited to, humans, non-human primates (monkeys, gorillas, chimpanzees, etc.), mice, and rats. In one aspect, a plurality of single cells derived from the same tissue or from adjacent tissues of the same individual are utilized. "Adjacent tissues" refers to, but are not limited to, tissues that are physically close to each other *in vivo* (e.g., small intestine and large intestine, and placenta and fetus), functionally related tissues (e.g., digestive system, respiratory system, and central nervous system).

The source of the RNA sequence data of the transcriptome of each single cell is not particularly limited. For example, data published in data banks such as DDBJ, EMBL-Bank/EBI, and GenBank/NCBI, and data published in literature such as NPL 36 can be used.

The source of the corresponding genome sequence data to be compared with the RNA sequence data of a transcriptome is not particularly limited. For example, data published in data banks such as DDBJ, EMBL-Bank/EBI, and GenBank/NCBI can be used.

The step of comparing the RNA sequence data of the transcriptome of each single cell with the corresponding genome sequence data and then screening for a site where the RNA sequence differs from the genome sequence can be performed using known software for comparing and mapping sequences.

For example, Burrows-Wheeler Aligner (BWA) can be used for mapping. BWA is a software package for mapping low-range sequences against a large reference genome, such as the human genome, and includes two algorithm types: BWA-backtrack, BWA-SW and BWA-MEM. Other software such as BarraCUDA, STAR, etc. may be used for mapping the RNA sequence data of a transcriptome to the corresponding genome sequence data.

In one aspect, an adapter sequence may be added to an RNA sequence for analysis. In that case, it is preferable to remove the adapter sequence prior to mapping the RNA sequence data to the genome sequence data.

Screening for a site where the RNA sequence differs from the genome sequence can be performed using known software for polymorphism detection and alignment display. For example, Samtools may be used. Samtools is software to be used for processing after short-read sequence alignment of DNA sequencing. Example of the main functions of Samtools include polymorphism detection, alignment display, indexing, data extraction, and file format conversion. In addition, software such as elPrep and Picard may be used for screening for a site where the RNA sequence differs from the genome sequence.

In step (2), of the sites identified by screening in step (1), a site where three or more single nucleotide polymorphisms are detected is screened for as a site of somatic mutation.

In the above invention, screening is performed for a site of somatic mutation. For example, according to Tomasetti et al. (NPL 8), the *in vivo* tissue-specific somatic mutation probability in normal lymphocytes, precursors of chronic lymphocytic leukemia (CLL), was 7.6 × 10⁻¹⁰ ±1.1 × 10⁻¹⁰ (SE) per nucleotide per cell division. Therefore, regarding stochastic somatic changes, an average of 3 mutations occur per cell division in the human genome, excluding repetitive regions (NPL 70 and NPL 71). The abundance of such somatic mutations makes it possible to generate cell lineages that trace back to the past even in normal tissues.

"Three single nucleotide polymorphisms" refers to three types of nucleotide polymorphisms. If a gene locus (here, a coordinate on the genome) is homozygous, whether a mutation occurred at the gene locus can be determined by comparison with a standard genome sequence. However, if a gene locus is heterozygous, it cannot be immediately determined whether it is the result of a mutation or is originally heterozygous. Specifically, when there are two mutation types, it is difficult to determine whether or not such mutations occurred at the relevant gene locus. However, in the case of three mutation types, this observation result cannot be explained without assuming at least one mutation. In the present invention, based on this logic, "site where three or more single nucleotide polymorphisms are detected" is targeted for mutation detection.

The upper limit of the number of polymorphisms in the "site where three or more single nucleotide polymorphisms are detected" is not particularly limited. For convenience of analysis, the number of polymorphisms should preferably not be too large. Without limitation, the number of polymorphisms is 15 or less, 12 or less, 10 or less, 8 or less, 6 or less, 5 or less, or 4 or less. In one aspect, the number of polymorphisms is three.

The "single nucleotide polymorphism" used in the present invention refers generally to a mutation that occurs at the single nucleotide level to another nucleotide (group), and may be any of nucleotide (group) substitution, insertion (addition), and deletion. "Mutation occurring at the single nucleotide level" includes not only single nucleotide mutation (point mutation) but also mutation occurring at multiple nucleotide units. In one aspect, the single nucleotide polymorphism is a single nucleotide mutation (point mutation). In one aspect, the single nucleotide polymorphism is a single nucleotide insertion and/or substitution. In one aspect, the single nucleotide polymorphism is a single nucleotide substitution.

In one aspect, in step (1), a site, for which the RNA sequence data of the transcriptome cannot be obtained in 50% or more of all cells, is presumed to have no mutation and is excluded from screening. The method utilizes RNA sequence data of a transcriptome. Unlike a static genome, the amount of transcripts (transcriptional products) fluctuates from cell to cell and over time. Therefore, there may be cases (sites) in which the RNA of a specific transcriptional product is not detected, and RNA sequence data cannot be obtained in some cells. In one aspect of the present invention, when the RNA sequence data of transcriptomes are obtained in 50% or more of all cells, that is, transcript sequences mapped to 50% or more cells are used for mutation detection. In this case, in step (1), a site, for which the RNA sequence data of the transcriptome cannot be obtained in 50% or more of all cells, is presumed to have no mutation and is excluded from screening. "Be presumed to have no mutation" means the use of the corresponding genome sequence in place thereof. "50% or more" of "in 50% or more of all cells" may be, without limitation, 55% or more, 60% or more, 65% or more, 70% or more, 75% or more, or 80% or more.

Due to the low coverage of transcripts (transcriptional products), potential mutation sites may be filtered out by the initial screening in some single cells. A threshold may be selected to exclude potential variants in the screening of the present invention. In examples herein, a threshold of 80% was used as an example solution. Specifically, a multiple mutation site observed in 80% or more of single cells tested was screened for. Without limitation, higher thresholds, such as a threshold of 90% or greater, or lower thresholds, such as a threshold of 60% or greater, or that of 70% or greater, may be employed.

In one aspect of the method, the somatic mutation to be screened for in step (2) is a low frequent somatic mutation.

The low frequent somatic mutation (minor allele) refers to a less frequent somatic mutation in transcripts observed in cell populations. The "allele" originally refers to an allele, but in the present specification, it may be used to extend the meaning to the one including single-nucleotide mutation. The concept of allele originally belongs to the genome sequence. However, assuming that there is a certain degree of correlation between the allele frequency due to the transcript sequence and the allele frequency due to the genome sequence, alleles with lower frequency in the genome sequence, that is, minor alleles are somatic mutation candidates to be screened for.

The assumption that "there is a certain degree of correlation between the allele frequency due to the transcript sequence and the allele frequency due to the genome sequence" is based on the empirical expectation that the allele frequency of the genome and the same of transcriptome reads are correlated (NPL 74 and NPL 75). However, it is preferable to further apply a more quantitative way of approach in order to reduce the error. For example, statistical haplotype phasing (NPL 76) is believed to be effective for improving estimates.

The frequency of a mutation found in alleles can be estimated, for example, by comparing the reference genome with the read sequence. The meaning of "low frequent" is, without limitation, for example, that the frequency is within 50%, preferably within 40%, within 30%, within 20%, within 10%, or within 5% of all mutations found in alleles from the least frequency. In one aspect, the meaning of "low frequent" is that the frequency of a mutation is within the first to the tenth, to the 8th, to the 5th, and to the 3rd lowest frequency among all mutations found in an allele from the lowest to the highest. In one aspect, the low frequent somatic mutation (minor allele) refers to a somatic mutation that occurs at the lowest frequency among all mutations found in an allele.

The implication of screening for a low frequent mutation (minor allele) is based on an assumption that a de novo somatic mutation in a cell is in the process of diffusing into the cell population and thus should be observed at a relatively low frequency. In the method, through selection of such low frequent mutations, de novo somatic mutations can be efficiently selected from a plurality of alleles based on a certain degree of correlation between the allele frequency of a transcript and the allele frequency of a DNA sequence. Therefore, it becomes possible to conduct phylogenetic analysis of cells more effectively.

In step (3), a cell lineage tree is generated based on the nucleotide sequence information of the site of somatic mutation obtained in step (2).

A cell lineage tree can be generated using known software for generating a lineage tree from sequence information. For example, one of software packages for generating a lineage tree (estimating the past) from gene sequence information, MEGA-X, can be used. Alternatively, software such as Phylip may be used.

Without limitation, the maximum parsimony method (NPL 46 and NPL 47) may be applied to the generation of a cell lineage tree. The maximum parsimony method is one of the method for generating a lineage tree by finding a mutation pattern that minimizes the number of mutations. Similar analysis can also be conducted using other methods such as the distance matrix method, the maximum likelihood method, and the Bayesian method.

The method may further include a step of estimating the cell type of each single cell from the cell lineage tree.

Estimation (identification) of the cell type of each cell from a cell lineage tree can be performed using known methods. For example, known tools such as principal component analysis, t-SNE, and UMAP may be used. Specifically, a gene expression matrix consisting of the types of single cells and the types of genes may be subjected to linear or nonlinear dimensionality reduction for clustering cells, or information on known marker genes may be used.

snpEff (NPL 45) is a tool for annotating predicted mutations and providing the same with information on their impact. It is possible to obtain information on the number of each mutation, positional information and types (SNP, insertion, deletion, etc.), and degree of influence (substitution to amino acid). A mutation site can be annotated based on the annotation assigned to the genome, depending on where in the genome the mutation site is located. However, since the data used in the method are transcript sequences, there may be some uncertainty. The SnpEff software can adopt annotations that are considered appropriate, in consideration of the circumstances. Furthermore, these annotations allow biological and evolutionary interpretation of each mutation.

In one aspect, the method may further include a step of comparing the information of the single cell type estimated from the gene expression profile of each single cell with the information of the cell lineage tree.

The source of the gene expression profile of each single cell is not particularly limited. For example, data published in databanks such as Expression Atlas and Gene Expression Omnibus, and data published in literature such as NPL 36 can be used.

In one aspect, the method can identify single cell types that could not be identified with the use of the gene expression profiles. In one aspect, gene expression profiles can be reviewed and corrected in terms of misidentified single cell types.

In one aspect, the method can be used to detect a somatic mutation associated with a disease or a symptom. The type of a disease or a symptom is not particularly limited. In one aspect, the disease or the symptom is a disease or a symptom caused by genetic mutation. Without limitation, the disease or the symptom is selected from the group consisting of cancer, dementia, cardiovascular disease, aging, autoimmune disease, neurodegenerative disease, and psychiatric disease.

### Significance of the present invention

The significance of the present invention will be described in a non-limiting manner for understanding the present invention. According to the present invention, a new framework is provided for estimating cell lineage trees using somatic mutations detected from covered single-cell transcriptomes. At this time, we have focused on the phylogenetic signature of single-cell transcriptomes rather than individual mutations. As a result, we have thus demonstrated that it is possible to reconstruct a cell lineage tree consistent with known biological knowledge. The significance of the present invention is to provide supporting information for inferring the overall lineage trees of single cells and interpreting gene expression profiles. In gene expression analysis, a dimensionality reduction method is often used. However, how to extract biologically relevant findings from the results obtained at the single-cell level is an unsolved problem (NPL 69). This framework allows us to interpret gene expression profiles from a different angle; that is, single-cell mosaicism.

Whereas pseudo-time course analysis uses heterodifferentiated cells in a single time snapshot, the real-time course that is an approach of the present invention utilizes somatic mutations, so as to be able to trace cell lineages back to descendant cells. Specifically, the method of the present invention makes it possible to estimate the time course of cells. "Time course estimation" is estimation about unobservable past events, etc., including, for example, the self-renewal stages of stem cells (Figs. 5c and 6a). Indeed, with the real-time course of the present invention, results that closely match those of the pseudo-time-course analysis can be obtained, and with the use of a completely different kind of data, namely mapped read sequences and their depths, results can be obtained by a method with finer granularity.

### 2. Programs, systems, etc. for conducting phylogenetic analysis of cells

In order to efficiently perform the method for conducting phylogenetic analysis of cells of the present invention, a program for automatically performing the method by a computer is preferably created. Also, this program may be stored on a storage medium, so as to be read by a computer. Furthermore, a dedicated system in which a program is stored in memory may be created, so that the program can be executed by a processor of a computer.

In one aspect, the present invention relates to a program for performing the method of the present invention.

In one aspect, the present invention is a program for performing a method for conducting phylogenetic analysis of cells, the method including:
(1) a step of comparing, for a plurality of single cells derived from the same individual, the RNA sequence data of the transcriptome of each single cell with the corresponding genome sequence data, and then screening for a site where the RNA sequence differs from the genome sequence;
(2) a step of screening the sites identified by screening in (1) for a site where three or more single nucleotide polymorphisms are detected, as a site of somatic mutation; and
(3) a step of generating a cell lineage tree based on the nucleotide sequence information of the site of somatic mutation obtained in (1).

In one aspect, the present invention relates to a storage medium, on which a program for performing the method of the present invention is stored.

In one aspect, the present invention relates to a system including a processor and a memory having a program stored therein that performs the method described in the method of the present invention when the program is executed by the processor.

In one aspect, the present invention relates to a system for performing phylogenetic analysis of cells, including:
(1) a means for comparing, for a plurality of single cells derived from the same individual, the RNA sequence data of the transcriptome of each single cell with the corresponding genome sequence data, and then screening for a site where the RNA sequence differs from the genome sequence;
(2) a means for screening the sites identified by screening in (1) for a site where three or more single nucleotide polymorphisms are detected, as a site of somatic mutation; and
(3) a means for generating a cell lineage tree based on the nucleotide sequence information of the site of somatic mutation obtained in (1).

The meanings, aspects and the like of "phylogenetic analysis of cells", "(1) comparing, for a plurality of single cells derived from the same individual, the RNA sequence data of the transcriptome of each single cell with the corresponding genome sequence data, and then screening for a site where the RNA sequence differs from the genome sequence", "(2) screening the sites identified by screening in (1) for a site where three or more single nucleotide polymorphisms are detected, as a site of somatic mutation", and "(3) generating a cell lineage tree based on the nucleotide sequence information of the site(s) of somatic mutation obtained in (1)" are as described in the section, "1. Method for conducting phylogenetic analysis of cells". All matters described in "1. Method for conducting phylogenetic analysis of cells" are also applied to systems, programs, and storage media.

### EXAMPLES

The present invention will be described in detail below based on examples, but the present invention is not limited to these examples. A person skilled in the art can easily make modifications and changes to the present invention based on the description of this specification, and they are included in the technical scope of the present invention.

### Example 1 Mapping of transcriptome sequence data and detection of mutations (1-1) Transcriptome data

As transcriptome data, two types of published transcriptome data obtained from normal (placental) tissue: SRP090944 batch 1 (54 cells) and batch 2 (33 cells) were used (NPL 36).

In NPL 36 (Pavlicev et al.) placental data from the perspective of cellular communication networks between two semiallogenic individuals, a mother and a fetus was analysed. In this literature, the intercellular interactome (NPL 37) was estimated from the gene expression of receptor-ligand pairs across cell types. As a result, cell type-specific expression of G protein-coupled receptors was found, suggesting that ligand-receptor profiles may be a reliable tool for cell type identification. The data have been registered in the DDBJ Sequence Read Archive (DRA) as SRS1732266 (SRX2225269)-SRS1732319 (SRX2225328).

Pavlicev et al., classifies single-cell data into 5 clusters (cell types), cytotrophoblast (CYT) 1, CYT2, CYT3, extravillous trophoblast (EVT), and maternal decidual cell (DC) by principal component analysis (PCA) on gene expression profiles and 300 marker genes. Regarding DC, Pavlicev et al., reports that these cells lack the expression of CD19, CD209, and CD163, but express ITGAX+/CD14+/CD4+/CD83+/CD86+ markers in combination, determining the cells are uterine dendritic cells. However, they did not observe the expression of some of genes characteristic of DCs, such as CLEC4C, THBD, CD1C, CD80, IL10, and IL12B.

### (1-2) Mapping of transcriptome sequence data

An overview of the data analysis pipeline of this example is shown in Fig. 1. Single-cell transcriptome sequence data were mapped to the human genome (GRCh38) (NPL 38 and NPL 39) using Burrows-Wheeler Aligner (BWA) 40 after removal of adapter sequences with trimomatic (NPL 41). Specifically, continuous processing was performed via an intermediate file by implementing a high-speed computer with these tools for creating a script for performing batch processing.

Batch 1 and batch 2 data were assumed not to share de devo mutations. Specifically, it was assumed that the two anonymous patients did not share a common (germline) mutation, i.e., they were not related to each other. Mutations were detected using Samtools (NPL 42). Specifically, through comparison between the reference genome and the read sequence, multiple alignments across cells were generated to estimate the positions and number of mutations.

In the examples of this specification, only single-nucleotide mutations were used, with the exception of all indel events detected. In addition, if incomplete site data were detected in 50% or more of all cells, that is, sites where the RNA sequence data of the transcriptome could not be obtained in 50% or more of all cells were presumed to have no mutation and excluded from screening.

### (1-3) Detection of single-cell transcriptome mutation

Clear mutations (deviations from the reference genome in mapped transcriptional products) can include both germline and somatic mutations. Since no information on haplotype phase was provided, a simple method for detecting somatic mutations at multiple repeat sites in cell populations was employed. Assuming that up to one somatic mutation occurs in one lineage, possible mutation patterns in the whole cell were classified (NPL 43 and NPL 44).

A conceptual diagram of one aspect of the method of the present invention is shown in Fig. 2. The method of the present invention includes the following steps.

(1) Comparing, for a plurality of single cells, the RNA sequence data of the transcriptome of each single cell with the corresponding genome sequence data, and then screening for a site where the RNA sequence differs from the genome sequence; and (2) Comparing the data of the site(s) identified by screening in step (1) between cells, and then screening for a site where three or more single nucleotide polymorphisms are detected, as a site of somatic mutation.

Two mutation sites alone are not sufficient to know whether the relevant mutation is a somatic mutation or a germline mutation, or whether the sites are heterozygous (Figs. 2a, 2b, 2d, 2g, and 2h). However, when multiple mutation sites, the number of which is three or more, are observed, it can indicate that at least one somatic mutation has occurred elsewhere in the cell lineage (Figs. 2c and 2e).

Meanwhile, in which lineage the mutation has occurred remains unknown. One idea is that when observed nucleotides share a reference site, the state of the reference genome can be used as the ancestral state. However, in our framework, polygenic sites are always heterozygous and reference genome data have not been haplotype phased. Here, we assumed that a minor allele is a newly derived nucleotide in the cell population, and determined to simply select the "minor allele" as a derived mutation. The selected minor allele is a somatic mutation that occurred at the lowest frequency among the mutations found in the alleles in this example.

The detected mutation sites were annotated using SnpEff software (NPL 45). Specifically, a VCF file obtained by mapping the transcript sequence to the genome was given as input data, and the annotation was added by specifying the genome data used.

### (1-4) Results

### Mapping of SRP090944 data (batch 1, 54 cells)

3,088,286,401-bp transcriptional product data of SRP090944 (batch 1: 54 cells) were mapped to the reference genome (GRCh38) (NPL 38 and NPL 39).

The average coverage rate (%) was 0.685% (SD: 0.231) (Fig. 4, cells of batch 1). The initial screening detected 1,965,629 sites that differed from the reference genome. In addition, the number of multiple mutation sites observed in all 54 single cells, where three or more types of nucleotides had been mutated, was 89. In addition, there were 2,083 multiple mutation sites observed in an average of 43.2 single cells (80% of 54 single cells). All data were quality controlled.

### SRP090944 data (batch 2, 33 cells)

The average coverage rate (%) was 0.477% (SD: 0.243) (Fig. 4, cells of batch 2). The first round of screening detected 830,905 sites that differed from the reference genome. The number of multiple mutation sites observed in all 33 single cells was 53. In addition, there were 574 multiple mutation sites observed in an average of 26.4 single cells (80% of 33 single cells).

### Annotation of mutation

Table 1 and Table 2 depict putative variants annotated by the SnpEff software (NPL 45).

Table 1 Putative variants by SnpEff software (SRP090944 data, batch 1, 54 cells)

**[Table 1]**

| Type | | | Region | | |
|---|---|---|---|---|---|
| Type (in alphabetical order) | Number | Percentage | Type (in alphabetical order) | Number | Percentage |
| 3_prime_UTR_variant | 1,214 | 16.18% | DOWNSTREAM | 557 | 7.43% |
| 5_prime_UTR_premature_start_codon_gain_variant | 6 | 0.08% | EXON | 991 | 13.22% |
| 5_prime_UTR_variant | 62 | 0.83% | INTERGENIC | 281 | 3.75% |
| downstream_gene_variant | 557 | 7.43% | INTRON | 3,901 | 52.04% |
| intergenic_region | 281 | 3.75% | SPLICE_SITE_ACCEPTOR | 2 | 0.03% |
| intron_variant | 3,905 | 52.05% | SPLICE_SITE_REGION | 16 | 0.21% |
| missense_variant | 550 | 7.33% | UPSTREAM | 466 | 6.22% |
| non_coding_transcript_exon_variant | 193 | 2.57% | UTR_3_PRIME | 1,214 | 16.20% |
| splice_acceptor_variant | 2 | 0.03% | UTR_5_PRIME | 68 | 0.91% |
| splice_region_variant | 16 | 0.21% | | | |
| start Jlost | 6 | 0.08% | | | |
| stop_gained | 45 | 0.60% | | | |
| synonymous_variant | 199 | 2.65% | | | |
| upstream_gene_variant | 466 | 6.21% | | | |

| | | | | | |
|---|---|---|---|---|---|
| Note: The number of mutations is not exclusive for each category. | | | | | |

Table 2 Putative variants by SnpEff software (SRP090944 data, batch 2, 33 cells)

**[Table 2]**

| Type | | | Region | | |
|---|---|---|---|---|---|
| Type (in alphabetical order) | Number | Percentage | Type (in alphabetical order) | Number | Percentage |
| 3_prime_UTR_variant | 886 | 15.89% | DOWNSTREAM | 515 | 9.26% |
| 5_prime_UTR_premature_start_codon_gain_variant | 2 | 0.04% | EXON | 455 | 8.19% |
| 5_prime_UTR_variant | 77 | 1.38% | INTERGENIC | 253 | 4.55% |
| downstream_gene_variant | 515 | 9.24% | INTRON | 2,968 | 53.39% |
| intergenic_region | 253 | 4.54% | SPLICE_SITE_DONOR | 12 | 0.22% |
| intron_variant | 2,980 | 53.45% | SPLICE_SITE_REGION | 32 | 0.58% |
| missense_variant | 216 | 3.87% | UPSTREAM | 359 | 6.46% |
| non_coding_transcript_exon_variant | 84 | 1.51% | UTR_3_PRIME | 886 | 15.94% |
| splice_donor_variant | 12 | 0.22% | UTR_5_PRIME | 79 | 1.42% |
| splice _region_variant | 32 | 0.57% | | | |
| stop_gained | 24 | 0.43% | | | |
| synonymous_variant | 135 | 2.42% | | | |
| upstream_gene_variant | 359 | 6.44% | | | |

| | | | | | |
|---|---|---|---|---|---|
| Note: The number of mutations is not exclusive for each category. | | | | | |

The results in Table 1 and Table 2 include alternative annotations such as nested intron genes (NPL 63), for example. Some mutation numbers overlap between categories. For example, 2,083 and 574 mutations were detected from the data of batch 1 and that of batch 2, respectively, on an 80% basis. However, the SnpEff software estimates 1,903 and 1,398 mutations from the data of batch 1 and that of batch 2, respectively, with the default parameter set. The fact that the orders of the numbers of both mutation sites estimated by independent software are almost the same indicates the validity of the annotation used in this analysis.

As a result of SnpEff, it was found that there were 550 and 216 missense mutations and 199 and 135 synonymous mutations from the data of batch 1 and that of batch 2, respectively.

### Example 2 Phylogenetic analysis of cells and generation of cell lineage tree

### (2-1) Phylogenetic analysis of cells

All observable mutation sites obtained in Example 1 were ligated to create a sequence alignment. "Mutation site ligation" refers to joining the sequence of a coding region in the vicinity of a mutation with a mutation site to create a 3-base codon in order to create a codon sequence containing the mutation. Cell lineage trees were reconstructed with default parameters using the maximum parsimony method (NPL 46 and NPL 47) implemented in MEGA X (NPL 48).

Specifically, the created multiple alignment was given to MEGA X as input data, and the cell lineage trees were reconstructed by selecting the maximum parsimony method from the GUI. The results were output in the Newick tree format (NPL 49) and used for subsequent processing.

### (2-2) Reanalysis of gene expression

For linear dimensionality reduction (LDR), principal component analysis (PCA) of gene expression patterns was performed using R (version 3.6.2) (NPL 54). Specifically, the gene expression pattern of a single cell is represented by a gene expression matrix consisting of the types of single cells and the types of genes. Of these, components in the direction of the types of genes were projected into the low dimensional space, so as to reduce the dimension of the gene expression matrix.

After that, t-SNE (NPL 32 and NPL 33) and UMAP (NPL 34 and NPL 35) were applied to perform nonlinear dimensionality reduction (NDR). The Louvain method (NPL 55) was used for clustering. Specifically, after performing global linear dimensional compression by principal component analysis, gene expression matrices were applied to each software as input data.

### (2-3) Comparative analysis of mutation patterns and gene expression profiles

Clustered cells were mapped to the cell lineage trees reconstructed based on cell genotypes. To that end, Phylogenetics for Mathematica (NPL 60) and Phylogenetics (NPL 61) were applied to develop Mathematica (NPL 59) code, AssignCluster2Cell.

"Mathematica" is formula manipulation software. "Phylogetics for Mathematica" is a library for formula manipulation software. "AssignCluster2Cell" is the name of a program developed to integrate the cell lineage trees and the gene expression profiles in this example.

AssignCluster2Cell loads the reconstructed cell lineage trees in Newick format, compares them with the pre-clustered cell population, and displays the degree of agreement on each node in a pie chart. A group of functions provided by Phylogetics for Mathematica and Phylogetics is used as a library for loading and visualizing the trees. AssignCluster2Cell itself is also a function written in Mathematica and optimized to run on Mathematica Notebook.

Using AssignCluster2Cell, the tree files in the Newick (standard data format for expressing phylogenetic trees) (NPL 62) format and the cluster table of the gene expression profiles were loaded, to associate the cluster IDs with the tree diagrams, and thus to evaluate the degree of monophyleticity (DoM) of each node in terms of the percentage of the number of clusters in its subtree.

The results are shown in Fig. 3. Each pie chart in Fig. 3 represents the DoM of each node. For example, it can be seen that if there is only one pie chart for node n, subtree n is completely monophyletic, and if there are two pie charts for node n', subtree n' is polyphyletic (subtree B in Fig. 3). Since cell type 2 predominates in subtree B, it is speculated that cell type 1 was derived from cell type 2. Thus, cell type associations were created based on gene expression profiles and cell lineages.

Theoretically, the root of a cell lineage tree represents a zygotic cell (fertilized egg), and its instance represents the zygotic genome of the fertilized egg. However, in the examples, the root of the estimated cell lineage tree may represent the progenitor cells of the observed cell population. Zygotic cells exist somewhere between the root of the cell lineage tree and the reference genome.

### (2-4) Cell lineage tree

Cell lineage trees of single cells of placental tissues of two different individuals were reconstructed (Figs. 5 and 6). A branch length represents the expected value of a mutation that has occurred on the branch because of the use of the maximum parsimony method (NPL 46 and NPL 47) performed by MEGA-X (NPL 48). Each lobe (operational taxonomic unit, OTU (NPL 64)) represents cells sampled from each tissue.

Cell lineages of cytotrophoblasts (CYTs: CYT1, CYT2, CYT3 in Figs. 3 and 4) and extravillous trophoblasts (EVTs) have been successfully modeled in the context of Notch/Wnt signaling (NPL 65). Specifically, EVTs differentiates from CYTs via cell column trophocytes (CCTs) (Fig. 9). The results of this example are consistent with the Notch/Wnt signaling model described in NPL 65. On the other hand, Pavlicev et al. state that their data include maternal decidual cells (DCs). However, the results of this example suggest that the putative DC cells are differentiated from fetal CYTs or CYT stem cells (Figs. 5 and 6). Further, reanalysis of transcriptome data using t-SNE (NPL 32 and NPL 33) also supported the correctness of the results of this example (Fig. 7).

Since somatic mutations essentially negatively affect cells to survive in cell populations, it is reasonable to assume that in normal tissues genuine somatic mutations have undergone purifying selection (NPL 50 and NPL 51). For example, the maximum likelihood method suggests that overall somatic mutations have undergone purifying selection, and the majority of detected somatic mutations are at least far from positive false.

On the other hand, in this example, a small number of positively selected sites were also detected in two samples collected from different individuals. This result may suggest that somatic mutations are also involved in genetic modifiers and play an important role.

Example 3 Evaluation of dN/dS ratio in coding region

In this example, the dN/dS ratio in a coding region was evaluated.

In normal tissues, genuine somatic mutations are expected to have undergone purifying selection (NPL 50 and NPL 51). To evaluate the detection reliability of the method of the present invention, the selective pressure of the variants detected in Example 1 was evaluated in terms of the dN/dS ratio. The codon sequences containing the detected variants were assembled to generate a codon alignment including the exon variants. The overall dN/dS ratio was calculated using Codeml in the Paml package (NPL 52).

Specifically, using the reconstructed cell lineage tree as a guide tree, a Palm environment was created with Anaconda, the necessary packages were implemented in it, and then the Palm Codeml module was invoked using Python code on Jupyter Notebook, thereby processing multiple alignments generated from codons. Further, paired dN/dS ratios between cell genotypes were also calculated using the Nei & Gojobori method (NPL 53). Specifically, the Codeml module was invoked to process multiple alignments generated from codons.

The overall dN/dS ratios of batch 1 and batch 2 of SRP090944 data were 0.865 and 0.556, respectively. This result suggests that somatic mutations in the two tissues underwent purifying selection.

On the other hand, in SRP090944 data batch 1, a mutation site that had undergone positive selection, Pr(ω>1)=1, was detected (NPL 66) by both Naive Empirical Bayes (NEB) analysis and Bayes Empirical Bayes (BEB) analysis where ω was the dN/dS ratio at a specific mutation site. This specific mutation sites is in NM-001144964.1, Homo sapiens NEDD4 like E3 ubiquitin protein ligase (NEDD4L), transcript variant b (NPL 67).

Moreover, in data batch 2 of SRP090944, a mutation site with Pr(ω>1) = 0.928 was found by NEB analysis and the same with 0.567 was found by BEB analysis. This mutation site is located at the gene locus of NM_001172895.1, Homo sapiens caveolin 1 (CAV1), transcript variant 2 (NPL 68).

Of all 1,485 and 561 possible pairs of cells in SRP090944 data batch 1 and batch 2, there were 482 and 71 lineages with a dN/dS ratio greater than 1, respectively. Thus, it was suggested that 67.5% and 87.3% of intercellular lineages were affected by purifying selection or neutrality, respectively.

### Comparative Example 1 Pseudo-Time Course Analysis

In this comparative example, using monocle3 (version 1.0.0) (NPL 56 to NPL 58) of R (version 4.1.2) (NPL 54), pseudo-time course analysis was conducted on SRP090944 data batch 1. Specifically, a single-cell gene expression matrix subjected to dimensional compression (26-dimension) was provided as input data, and then a relative pseudo-time expected to reflect differentiation aspects was assigned to each single cell.

Mathematica ListContourPlot function was used to visualize the results (NPL 59). Specifically, relative temporal contour lines were obtained by tertiary interpolation of single cells projected onto a two-dimensional plane at the assigned pseudo-time.

The results are shown in Fig. 8. Cellular pseudo-time is represented by contour lines generated by Mathematica ListContourPlot function (NPL 59) with interpolation order 3. The results were fairly consistent with the results obtained using the method of the present invention, except for the direction of pseudo-time. For example, the number of cells with a pseudo time greater than 2.5 is 16 (SRR4371527, SRR4371531, SRR4371532, SRR4371533, SRR4371536, SRR4371542, SRR4371547, SRR4371563, SRR4371566, SRR4371568, SRR4371569, SRR4371570, SRR4371571, SRR4371572, SRR4371575 and SRR4371577). Ten of them are directly derived from the self-renewal stage of putative stem cells as CYT cells according to our analysis (Fig. 5c).

### Evaluation of Examples

Examples demonstrated that somatic cell lineage trees of human placental tissues can be reconstructed using low-pass single-cell RNA sequence data. Such lineage trees are consistent with the known placental cell lineages for four cell types: cytotrophoblast (CYT) I, CYT II, CYT III, and extravillous trophoblast (EVT). Unlike pseudo-time course analysis using heterogeneously differentiated cells in a single time-snap, the method of the present invention uses somatic mutations to trace cell lineages back to descendant cells. Presumed descendant cells are therefore represented as internal nodes (vertices) of the reconstructed cellular trajectory tree.

The obtained "phylogenetic" signature of the cells was consistent with a model system integrating the role of Notch/Wnt signaling in villous stem cell and extravillous cell differentiation in placenta sampled from two individuals. This suggests that a cell lineage tree can be retrospectively estimated even with low-pass sequence data. The detection of individual somatic mutations has been considered to be difficult unless the use of ultra-deep sequencing, but the present invention enables such detection. It was also discovered that cells with somatic mutations have undergone purifying selection as expected, but the adaptive evolution signal is present at least at certain sites.

Further, the quality of the detected somatic mutations was evaluated in an evolutionary framework. It was confirmed that the detected somatic mutations had undergone purifying selection (dN/dS<1) as a whole, but also exhibited signs of adaptive evolution (dN/dS>1), at least at certain sites. These evolutionary insights support moderate reliability of the results of the present invention, at least in the coding region. Furthermore, the results of the examples were consistent with the results of pseudo-time course analysis using completely different types of data including the sequences of mapped reads and depths thereof.

### INDUSTRIAL APPLICABILITY

In single-cell gene expression analysis, low-pass sequence data (transcriptome RNA sequence data) is a "by-product", and polymorphism (mosaic) information between single cells has often been ignored. The present invention has demonstrated that it is possible to extract significant genotype information by a gleaning-like method. The somatic mutational analysis of single-cell transcriptome data of the present invention enables the interpretation of high-dimensional gene expression data. Moreover, the biological significance of somatic mutation sheds light on a new perspective of "evolution" in an individual.

## Claims

1. A method for conducting phylogenetic analysis of cells, comprising steps of:
(1) comparing, for a plurality of single cells derived from the same individual, the RNA sequence data of the transcriptome of each single cell with the corresponding genome sequence data, and then screening for a site where the RNA sequence differs from the genome sequence;
(2) screening the sites identified by screening in step (1) for a site where three or more single nucleotide polymorphisms are detected, as a site of somatic mutation; and
(3) generating a cell lineage tree based on the nucleotide sequence information of the site of somatic mutation obtained in step (2).

2. The method according to claim 1, further comprising a step of (4) estimating the cell type of each single cell from the cell lineage tree.

3. The method according to claim 1 or 2, further comprising a step of comparing the information of the single cell type estimated from the gene expression profile of each single cell with the information of the cell lineage tree.

4. The method according to any one of claims 1 to 3, wherein the single nucleotide polymorphism is a single nucleotide substitution.

5. The method according to any one of claims 1 to 4, wherein in step (1), a site, for which the RNA sequence data of the transcriptome cannot be obtained in 50% or more of all cells, is presumed to have no mutation and is excluded from screening.

6. The method according to any one of claims 1 to 5, wherein the somatic mutation to be screened for in step (2) is a low frequent somatic mutation.

7. The method of any one of claims 1 to 6, for detecting a somatic mutation associated with a disease or a symptom.

8. The method according to claim 7, wherein the disease or the symptom is selected from the group consisting of cancer, dementia, cardiovascular disease, aging, autoimmune disease, neurodegenerative disease, and psychiatric disease.

9. A program for performing the method according to any one of claims 1 to 8.

10. A storage medium, on which a program for performing the method according to any one of claims 1 to 8 is stored.

11. A system comprising a processor and a memory having a program stored therein that performs the method according to any one of claims 1 to 8 when the program is executed by the processor.
